# EUROPÄISCHE PATENTANMELDUNG

(11) **EP 1 344 507 A1**
(43) Veröffentlichungstag der Anmeldung: **17.09.2003**
(21) Anmeldenummer: 02005631.3
(22) Anmeldetag: 12.03.2002
(51) Int. Cl.: A61F 2/44

(54) **Zwischenwirbelprothese für die Halswirbelsäule**

(71) Anmelder: Waldemar Link (GmbH & Co.), 22339 Hamburg (DE)
(72) Erfinder: Keller, Arnold, 23863 Kayhude (DE); McAfee,Paul C,M.D,c/o Scoliosis and Spine Center, Baltimore, MD 21204 (US)
(74) Vertreter: Glawe, Delfs, Moll & Partner

(57) **Zusammenfassung**

Zwischenwirbelprothese für die Halswirbelsäule mit zwei jeweils mit einem Wirbel zu verbindenden Deckplatten (1, 2) und einem Prothesenkern (10), der von einem an einer Deckplatte (1) angeordneten Sitz (6 bis 9) gegen seitliche Verschiebung gesichert ist und eine mit der anderen Deckplatte 2 zusammenwirkende Gleitgelenkfläche (25) bildet. Die Gleitgelenkfläche (25) erstreckt sich im wesentlichen über die gesamte Prothesenquerschnittsausdehnung. Gegen Entweichen aus dem Deckplattenzwischenraum wird sie dadurch geschützt, daß der Sitz (6 bis 9) den Prothesenkern (10) nicht nur gegen seitliche Verschiebung, sondern auch gegen Abheben sichert.

## Beschreibung

Zwischenwirbelprothesen dienen zum Ersatz der Bandscheibe. Für die Lendenwirbelsäule sind Zwischenwirbelprothesen entwickelt worden, die aus zwei jeweils mit einem Wirbel zu verbindenden Deckplatten und einem Prothesenkern bestehen, der von einem an einer Deckplatte angeordneten Sitz gegen seitliche Verschiebung gesichert ist und eine mit der anderen Deckplatte zusammenwirkende Gleitgelenkfläche bildet (EP-B-471 821, DE-C-30 23 353). Dabei besteht der Sitz, der den Prothesenkern an der unteren Deckplatte festhält, aus einer ebenen Auflagefläche und einem diese umgebenden, hochstehenden Rand. Der Rand muß so hoch sein, daß er auch bei maximaler Beugung und daraus folgender Öffnung der Deckplatten auf der Rückseite des Gelenks das Entweichen des Kerns aus der Prothese sicher verhindert. Denn es muß jedenfalls vermieden werden, daß der Kern in den Rückenmarksbereich gelangt. Damit der Rand diese Aufgabe erfüllen kann, ist er verhältnismäßig großräumig ausgebildet. Dies geht auf Kosten der Flächenausdehnung der Gleitfläche am Prothesenkern. Dies ist im Bereich der Lendenwirbelsäule unschädlich, weil das Kraftaufnahmevermögen der für den Kern verwendeten Werkstoffe (beispielsweise hochdichtes Polyethylen) hinreichende Festigkeit aufweist.

Im Bereich der Halswirbelsäule stellt sich das Problem der sicheren Zurückhaltung des Prothesenkerns in noch höherem Maße, weil die kritischen Bereiche näher beieinander liegen. Gleichzeitig ist die Flächenausdehnung des Wirbelbereichs, der für die Aufnahme der Prothese zur Verfügung steht, viel kleiner. Es ist deshalb nicht möglich, die Erfahrungen, die bei Prothesen für die Lendenwirbelsäule gesammelt wurden, einfach auf Prothesen für die Halswirbelsäule zu übertragen.

Der Erfindung liegt daher die Aufgabe zugrunde, eine Zwischenwirbelprothese für die Halswirbelsäule zu schaffen, die einerseits eine hinreichende Kraftübertragungsfläche in dem Prothesengelenk zur Verfügung stellt und andererseits den Prothesenkern sicher in der Prothese zurückhält.

Die erfindungsgemäße Lösung besteht in den Merkmalen des Anspruchs 1 und vorzugsweise denen der Unteransprüche. Danach ist vorgesehen, daß die Gleitgelenkfläche des Prothesenkerns sich im wesentlichen über die gesamte Prothesenquerschnittsausdehnung erstreckt. Da dies die Sicherung des Prothesenkerns durch einen hochstehenden Rand ausschließt, ist der den Prothesenkern an einer Deckplatte festhaltende Sitz so ausgebildet, daß er ihn gegen Abheben sichert. Dadurch ist Sorge dafür getragen, daß er nicht über die ihn gegen seitliche Verschiebung sichernden Teile des Sitzes hinweggleiten kann, auch wenn diese verhältnismäßig niedrig ausgebildet sind.

Die den Prothesenkern festhaltenden Teile des Sitzes sind zweckmäßigerweise als eine von ventral her offene Einschubführung für den Prothesenkern ausgebildet, damit er gewünschtenfalls nach der Implantation der Deckplatten in die Prothese eingefügt werden kann. Wenn die Prothese als Ganzes implantiert wird, kann die Halterung des Kerns an der Deckplatte auch anders ausgeführt sein.

Die Einschubführung besteht zweckmäßigerweise aus zwei lateral angeordneten, sich im wesentlichen in AP-Richtung erstreckenden, hinterschnittenen Randleisten an einem der beiden Teile (Deckplatte bzw. Kern) und Vorsprüngen am anderen Teil, die in den Hinterschnitt der Randleisten eingreifen. Diese Vorsprünge am anderen Teil können - wie bei einer Schublade - ebenfalls leistenförmig sein. Beispielsweise können die hinterschnittenen Randleisten lateral an den gegenüberliegenden Seitenrändern der unteren Deckplatte der Prothese vorgesehen sein, während an den entsprechenden Rändern des Prothesenkerns eine Nut eingeschnitten ist, die die Randleisten aufnimmt, wobei die Nut wiederum einen leistenförmigen Vorsprung aufweist, der in den Hinterschnitt der deckplattenseitigen Randleisten eingreift. Solche leistenförmig zusammenwirkenden Teile der Einschubführung müssen nicht am äußeren Rand der Deckplatte bzw. des Prothesenkerns angeordnet sein; sie können vielmehr auch im Zentrum vorgesehen sein, beispielsweise als Nut in der Mitte des Prothesenkerns, deren Ränder die hinterschnittenen Randleisten bilden und die mit einem entsprechenden leistenförmigen Vorsprung der unteren Deckplatte zusammenwirkt.

Wenn die Führungsorgane an der Deckplatte und am Prothesenkern beiderseits leistenförmig sind, ergibt sich eine Richtungsstabilität des Prothesenkerns, die z.B. dann erwünscht sein kann, wenn er auf der unteren Deckplatte um eine gewisse Strecke in AP-Richtung beweglich bleiben soll. In anderen Fällen kann es erwünscht sein, daß der Prothesenkern gegenüber der unteren Deckplatte rotativ beweglich ist. In diesen Fällen werden die Führungsorgane der Einschubführung an einem der beiden Teile (Deckplatte bzw. Kern) kreisbogenförmig ausgebildet. Beispielsweise kann die Einschubführung an der unteren Deckplatte durch zwei einander gegenüberstehende, parallele Randleisten gebildet sein, während die damit zusammenwirkenden Vorsprünge von einem kreisförmig oder teilweise kreisförmig begrenzten Vorsprung gebildet sind. Die drehbare Variante des Prothesenkerns ist insbesondere dann von Interesse, wenn man eine Gleitflächenform wählt, die unterschiedliche Radien im Frontal- und Medianschnitt hat, die der Erzielung unterschiedlicher Beugeeigenschaften nach vorne und zu den Seiten hin dienen sollen.

Am dorsalen Ende ist die Einschubführung durch einen festen Anschlag begrenzt, der das Heraustreten des Prothesenkerns zum Rückenmarkkanal hin verhindert. Auch auf der Vorderseite kann ein Anschlag angeordnet sein, der jedoch beweglich sein soll, damit der Kern ohne weiteres in die Einschubführung hinein geschoben werden kann. Vorzugsweise handelt es sich um einen Rastanschlag, der von einem an der Deckplatte angeordneten festen Anschlag und einem an dem Prothesenkern fest angeordneten, aufgrund der Flexibilität des Prothesenkerns nachgiebigen Anschlagteil gebildet ist. Wenigstens einer der beiden Anschlagteile sollte dabei auf seiner der Anschlagseite gegenüberliegenden Seite eine schräge Auflauframpe für den anderen Anschlagteil bilden.

Bei einer anderen Ausführung des beweglichen Anschlags umfaßt dieser einen Anschlagteil, der zwischen einer sperrenden und einer nicht sperrenden Stellung umstellbar ist.

Die Erfindung wird im folgenden näher unter Bezugnahme auf die Zeichnung erläutert, die ein vorteilhaftes Ausführungsbeispiel veranschaulicht. Es zeigen:
- Fig. 1: einen Frontalschnitt und
- Fig. 2: einen Medianschnitt durch eine erste Ausführungsform der Prothese,
- Fig. 3: eine Zerlegungsdarstellung der ersten Ausführungsform der Prothese,
- Fig. 4: die untere Deckplatte und den Prothesenkern der ersten Ausführungsform in perspektivischer Darstellung im montierten Zustand,
- Fig. 5: eine zweite Ausführungsform der Prothese,
- Fig. 6: eine Variante der unteren Deckplatte der zweiten Ausführungsform und
- Fig. 7: einen der Fig. 2 entsprechenden Medianschnitt durch eine dritte Ausführungsform.

Die untere Deckplatte 1 und die obere Deckplatte 2 der ersten Ausführungsform weisen Oberflächen 3 bzw. 4 auf, die zur Verankerung am zugehörigen Wirbelkörper bestimmt sind. Sie sind vorzugsweise eben. Es sind aber auch andere, im wesentlichen flache Gestaltungen einschließlich geeigneter Oberflächenstrukturen zur besseren Verankerung am Knochen denkbar. Die Deckplatten bestehen vorzugsweise aus Metall.

Die untere Deckplatte 1 wendet der oberen Deckplatte 2 eine ebene Oberseite 5 zu, die auf drei Seiten von einem Kragen 6 eingefaßt ist, der oberhalb eines inneren Hinterschnitts eine nach innen ragende Leiste 7 bildet. Die untere Deckplatte 1 ist in der Draufsicht etwa rechteckig mit abgerundeten Ecken oder oval mit größerer Abmessung in Querrichtung als in AP-Richtung gestaltet. Die seitlichen Abschnitte 8, 9 des Kragens 6 verlaufen zueinander parallel und geradlinig.

Die Oberfläche 5 und der Kragen 6 der unteren Deckplatte bilden einen Sitz für den Prothesenkern 10, der aus gleitgünstigem Werkstoff, beispielsweise Polyethylen, besteht. Er hat eine zur Fläche 5 passende ebene Unterfläche, die zumindest an den Seiten von einer Randleiste 11 umgrenzt wird, oberhalb welcher sich eine Nut 12 befindet. Die Leiste 11 greift im montierten Zustand in den Hinterschnitt des Kragens 6 unterhalb der Leiste 7. Die Leiste 7 greift in die Nut 12 ein. Dadurch ist der Prothesenkern gegen Abheben von der unteren Deckplatte gesichert.

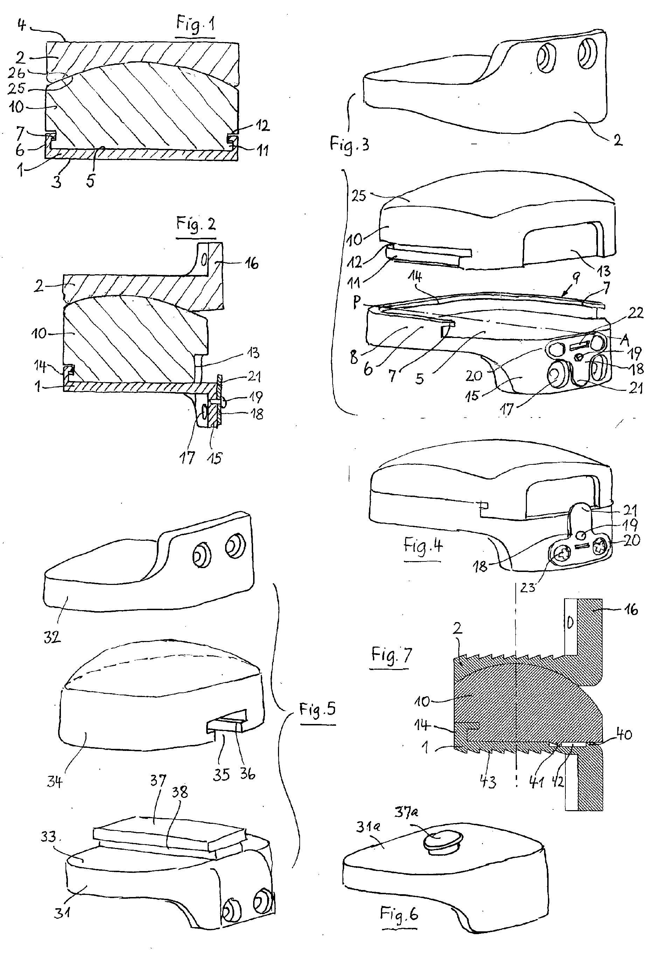

Die seitlichen Abschnitte 8, 9 des Kragens 6 bilden eine Führung für den Prothesenkern, in welche dieser von der offenen, ventralen Seite (in Fig. 2 rechts) her eingeschoben werden kann. Dieser Gedanke verdient Schutz unabhängig von der Flächenausdehnung der Gleitfläche. Damit er leicht eingeschoben werden kann, ist zweckmäßigerweise zwischen dem Kragen 6 und dem Rand des Kerns 10 Gleitspiel vorhanden. Die seitlichen Abschnitte 8, 9 des Kragens 6 sind auf der dorsalen Seite (in Fig. 2 links) durch einen Abschnitt 14 des Kragens 6 verbunden, der dieselbe Querschnittsgestalt wie die seitlichen Abschnitten 8, 9 haben kann; erforderlich ist dies jedoch nicht. Der dorsale Abschnitt 14 des Kragens 6 wirkt als Sicherungsanschlag, der ein Entweichen des Kerns 10 in dorsaler Richtung aus dem Zwischenraum zwischen den Deckplatten 1 und 2 verhindert.

An ihrem ventralen Rand weisen die Deckplatten 1, 2 je einen rechtwinklig von ihnen abragenden Flansch 15, 16 auf, der Schraubenlöcher 17 zur Befestigung am Wirbelkörper enthält. Am Flansch 15 der unteren Deckplatte 1 ist mittig zwischen zwei Schraubenlöchern 17 eine Sicherungsplatte 18 mittels eines Kopfbolzens 19 drehbar befestigt. Sie weist zwei sich im wesentlichen zur Seite hin erstreckende Flügel 20 und eine sich quer dazu erstreckende Zunge 21 auf. Sie besteht aus federndem Metall und ist so vorgespannt, daß ihre Flügel 20 gegen die untere Deckplatte drücken. Zum Eingriff eines Drehwerkzeugs, beispielsweise eines Schraubenziehers, enthält sie eine passende Öffnung oder Vertiefung 22. Wenn sie sich in der in Fig. 3 dargestellten Montagestellung befindet, ragt sie nicht über die Oberfläche 5 der unteren Deckplatte hinaus. Der Kern kann daher unbehindert in die von dem Kragen 6 gebildete Einschubführung eingeschoben werden. Wird sie um 180° gedreht, wie es in Fig. 2 und 4 dargestellt ist, ragt die Zunge 21 über die Oberfläche 5 und bildet dadurch einen Anschlag, der den Kern 10 am Verlassen der Führung zur ventralen Seite hin hindert.

In der in Fig. 3 gezeigten Montagestellung läßt die Sicherungsplatte 18 die Schraubenlöcher 17 für die Montage der Befestigungsschrauben frei. Wie in Fig. 4 gezeigt, überdecken die Flügel 20 in der Sicherungsstellung ganz oder teilweise die Schraubenlöcher 17 und drücken elastisch auf die darin befindlichen Schraubenköpfe, um sie am Heraustreten aus den Schraubenlöchern 17 zu hindern. Sie enthalten eine Öffnung, die sich unter der Vorspannung einrastend über die Kuppen der in Fig 4 angedeuteten Schraubenköpfe 23 legt und dadurch die Sicherungsplatte am Verlassen der Sicherungsstellung hindert.

Zwischen der als Anschlag für den Prothesenkern 10 vorgesehenen Zunge 21 der Sicherungsplatte 18 und der ihr gegenüberstehenden Fläche 13 des Kerns 10 befindet sich - wie in Fig. 2 gezeigt - ein Freiraum von wenigen Millimetern. Dadurch kann sich der Kern 10 in der von den seitlichen Abschnitten 8, 9 des Kragens 6 gebildeten Führung um eine gewisse Strecke in AP-Richtung (die in Fig. 3 strichpunktiert angedeutete ist) bewegen. Wenn dieses Bewegungsspiel nicht gewünscht wird, reduziert man den Freiraum zwischen der Fläche 13 und dem Anschlag 14 auf Null.

Oberseitig weist der Kern 10 eine vorzugsweise konvex-sphärische Gelenkfläche 25 auf, die zur Bildung eines Gelenks zusammenwirkt mit der unterseitigen, konkav-sphärischen Gleitfläche 26 der oberen Deckplatte 2. Der Kern hat allseits im wesentlichen dieselbe Umrißgestalt in der Draufsicht wie die untere Deckplatte 1 und die obere Deckplatte 2. Er überdeckt insbesondere den Kragen 6, so daß die Größe der von dem Kern zur Verfügung gestellten Gleitfläche 25 durch das Vorhandensein des Kragens 6 nicht verringert wird. Der Kragen 6 kann im Verhältnis zur Höhe des Kerns 10 klein ausgebildet sein. Trotzdem kann der Kern dem Zwischenraum zwischen den Deckplatten 1 und 2 nicht entweichen, weil er durch das Zusammenspiel der hinterschnittenen Leisten 7 und 11 am Abheben von der Deckplatte 1 gehindert wird.

In der zweiten Ausführungsform gemäß Fig. 5 besteht die Prothese aus einer unteren Deckplatte 31 und einer oberen Deckplatte 32 sowie einem Kern 34. Die untere Deckplatte weist eine obere, ebene Fläche 33 auf, auf der der Prothesenkern 34 aufliegt. Während dieser Kern in der ersten Ausführungsform an seinen Außenseite geführt ist, besitzt er in der zweiten Ausführungsform eine langgestreckte Ausnehmung 35 mit hinterschnittenen Seitenrändern 36, die mit einem langgestreckten Vorsprung 37 der unteren Deckplatte mit entsprechend hinterschnittenen Rändern 38 zusammenwirken. Der Kern 34 wird dadurch - ebenso wie unter Bezugnahme auf das erste Ausführungsbeispiel erläutert - in AP-Richtung beweglich gegenüber der unteren Deckplatte 31 geführt. Außerdem ist er durch die zusammenwirkenden Hinterschnitte gegen ein Anheben von der unteren Deckplatte geschützt. Es können geeignete (nicht gezeigte) Anschläge vorgesehen sein, die das Herausrutschen des Prothesenkerns aus dem Plattenzwischenraum verhindern.

Die untere Deckplatte 31 kann durch die in Fig. 5 dargestellte untere Deckplatte 31a ersetzt werden, die sich von der unteren Deckplatte 31 dadurch unterscheidet, daß ihr Vorsprung 37a nicht langgestreckt, sondern in der Draufsicht kreisförmig begrenzt ausgeführt ist. Dies ermöglicht dem Prothesenkern 34 eine Rotation um den Vorsprung 37a, ohne dessen gewünschte AP-Bewegung zu behindern. Eine solche Rotierbarkeit des Kerns gegenüber der unteren Deckplatte kann erwünscht sein , wenn die Gleitflächen 25, 26 des Prothesenkerns und der oberen Deckplatte nicht sphärisch, sondern mit unterschiedlichen Krümmungsradien in den Median- und Frontalebenen ausgestattet sind (beispielsweise ellipsoidisch). Während bei sphärischer Ausbildung der Gleitflächen diese eine Rotationsbewegung zwischen der oberen Deckplatte und dem Prothesenkern ermöglichen, kann diese Rotationsbewegung bei asphärischer Ausbildung der Gleitflächen dank einer Ausführung der unteren Deckplatte gemäß Fig. 6 zwischen den Prothesenkern und der unteren Deckplatte stattfinden.

Eine solche Rotierbarkeit des Kerns gegenüber der unteren Deckplatte kann auch bei der Ausführungsform nach Fig. 1 bis 3 vorgesehen werden, indem der Rand 11, 12 des Kerns 10 kreisförmig gestaltet wird. Eine ganz oder teilweise kreisförmige Gestaltung des Kragens 6 der unteren Deckplatte 1 ist damit nicht zwangsläufig verbinden, obgleich sie möglich ist.

Soweit im folgenden nicht anders erläutert, gleicht die dritte Ausführungsform gemäß Fig. 7 der ersten Ausführungsform. Der wesentliche Unterschied besteht darin, daß statt eines beweglichen Anschlags ein Rastanschlag vorgesehen ist. Dieser besteht aus einem Anschlagteil 40, der von der unteren Deckplatte 1 hochragt, und einem von der Unterfläche des Prothesenkerns 10 nach unten vorspringenden, fest damit verbundenen Anschlagteil 41, die einander Anschlagflächen zuwenden. Ihre davon abgewendeten Seiten sind als schräge Auflauframpen zur Erleichterung der Montage ausgebildet. Hinter dem Anschlagteil 40 ist in die Platte 1 eine Vertiefung 42 eingearbeitet, deren Breite und Tiefe nicht wesentlich größer ist als die Breite und Höhe des kernseitigen Anschlagteils 41. Dank dieser Vertiefung 42 kann der Kern 10 frei in die von den seitlichen Abschnitten 8, 9 des Kragens 6 gebildete Einschubführung der unteren Deckplatte eingeschoben werden, ohne durch den Anschlagteil 40 behindert zu werden. Sobald die Schrägflächen 43, 44 miteinander in Kontakt kommen, bedarf es eines gewissen Einschubdrucks, um die Anschlagteile 40, 41 unter entsprechender elastischer Verformung des Kerns 10 übereinander weg zu heben, bis ihre Anschlagflächen hintereinander einrasten. Im dargestellten Beispiel haben die Anschlagflächen Abstand in AP-Richtung voneinander, wenn der Kern ganz nach dorsal auf die Platte 1 aufgeschoben ist. Um die Strecke dieses Abstands hat er gegenüber der Platte 1 Bewegungsfreiheit nach ventral. Wenn eine solche AP-Beweglichkeit nicht erwünscht ist, werden die Anschläge so angeordnet, daß ihr Anschlagflächen im montierten Zustand im wesentlichen spielfrei aneinander anliegen.

Fig 7 zeigt ferner die Möglichkeit, die Sicherheit des Prothesensitzes zwischen den Wirbelkörpern dadurch zu vergrößern, daß sägezahnförmige Querrippen 43 auf den Außenflächen der Deckplatten vorgesehen werden, die einer Relativbeweung der Prothese nach ventral Widerstand entgegensetzen.

## Patentansprüche

1. Zwischenwirbelprothese für die Halswirbelsäule mit zwei jeweils mit einem Wirbel zu verbindenden Deckplatten (1, 2, 31, 32) und einem Prothesenkern (10, 34), der von einem an einer Deckplatte (1) angeordneten Sitz gegen seitliche Verschiebung gesichert ist und eine mit der anderen Deckplatte (2, 32) zusammenwirkende Gleitgelenkfläche (25) bildet, **dadurch gekennzeichnet, daß** die Gleitgelenkfläche (25) sich im wesentlichen über die gesamte Prothesenquerschnittsausdehnung erstreckt und der Sitz den Prothesenkern gegen Abheben sichert.

2. Prothese nach Anspruch 1, **dadurch gekennzeichnet, daß** ein von ventral her offene Einschubführung (7, 11, 12) für den Prothesenkern (10) vorgesehen ist.

3. Prothese nach Anspruch 2, **dadurch gekennzeichnet, daß** die Einschubführung von zwei lateral angeordneten, sich im wesentlichen in AP-Richtung erstreckenden, hinterschnittenen Randleisten (7) an einem der beiden Teile (Deckplatte 1, 31 bzw. Kern 10, 34) und Vorsprüngen (11, 36) am anderen Teil gebildet sind, die in den Hinterschnitt der Randleisten (7) eingreifen.

4. Prothese nach Anspruch 3, **dadurch gekennzeichnet, daß** die Vorsprünge (11, 36) am anderen Teil (10, 34) ebenfalls leistenförmig sind.

5. Prothese nach Anspruch 3, **dadurch gekennzeichnet, daß** die Vorsprünge (37a) an dem anderen Teil (31a) kreisbogenförmig sind.

6. Prothese nach einem der Ansprüche 3 bis 5, **dadurch gekennzeichnet, daß** die hinterschnittenen Randleisten (7) an der Deckplatte (1) angeordnet sind.

7. Prothese nach einem der Ansprüche 3 bis 6, **dadurch gekennzeichnet, daß** ein das dorsale Austreten des Prothesenkerns (10) aus der Einschubführung (7) hindernder, fester Anschlag (14) vorgesehen vorgesehen ist.

8. Prothese insbesondere nach Anspruch 7, **dadurch gekennzeichnet, daß** der Anschlag (21) beweglich ist.

9. Prothese nach Anspruch 7, **dadurch gekennzeichnet, daß** der Anschlag ein Rastanschlag (40, 41) ist.

10. Prothese nach Anspruch 9, **dadurch gekennzeichnet, daß** der Rastanschlag von einem an der Deckplatte (1) angeordneten festen Anschlagteil (40) und einem an dem Prothesenkern (10) fest angeordneten, aufgrund der Flexibilität des Prothesenkerns nachgiebigen Anschlagteil (41) gebildet ist.

11. Prothese nach Anspruch 10, **dadurch gekennzeichnet, daß** wenigstens einer der beiden Anschlagteile (40, 41) auf seiner der Anschlagseite gegenüberliegenden Seite eine schräge Auflauframpe für den anderen Anschlagteil aufweist.

12. Prothese nach Anspruch 8, **dadurch gekennzeichnet, daß** der bewegliche Anschlag an der Deckplatte (1) einen zwischen einer sperrenden und einer nicht sperrenden Stellung umstellbaren Anschlagteil (21) umfaßt.

13. Prothese nach Anspruch 12, **dadurch gekennzeichnet, daß** der bewegliche Anschlag (21) mit einer beweglichen Schraubensicherung (20) verbunden ist.

14. Prothese nach Anspruch 5, **dadurch gekennzeichnet, daß** die von dem Prothesenkern (10) gebildete Gelenkfläche (25) im Frontalschnitt einen größeren Krümmungsradius als im Medianschnitt hat.
